Europäisches Patentamt

European Patent Office

Office européen des brevets

(11) Veröffentlichungsnummer: **0 363 489**
**A1**

## (12) EUROPÄISCHE PATENTANMELDUNG
### veröffentlicht nach Art. 158 Abs. 3 EPÜ

(21) Anmeldenummer: 88907442.3

(22) Anmeldetag: 18.04.88

(86) Internationale Anmeldenummer:
PCT/SU88/00083

(87) Internationale Veröffentlichungsnummer:
WO 89/10354 (02.11.89 89/26)

(51) Int. Cl.⁵: C07D 207/27

(43) Veröffentlichungstag der Anmeldung:
18.04.90 Patentblatt 90/16

(84) Benannte Vertragsstaaten:
BE CH DE FR GB IT LI

(71) Anmelder: 2-i MOSKOVSKY
GOSUDARSTVENNY MEDITSINSKY INSTITUT
IMENI N.I. PIROGOVA
ul. Ostrovityanova 1
Moscow, 117437(SU)

Anmelder: NAUCHNO-PROIZVODSTVENNOE
OBIEDINENIE "BIOLAR" AKADEMII NAUK
SSSR
Latviiskaya SSR
Olaine, 229014(SU)

(72) Erfinder: SHIPOV, Alexandr Gennadievich
ul. Molodogvardeiskaya, 4-86
Moscow, 121467(SU)
Erfinder: KRAMAROVA, Evgenia Petrovna
ul. Z.Kosmodemyanskoi, 17-2-19
Moskovskaya obl. Schelkovo, 141100(SU)
Erfinder: BAUKOV, Jury Ivanovich
ul. Akademika Anokhina, 34-2-253
Moscow, 117602(SU)
Erfinder: ZIEMELIS, Kristap Martynovich
ul. Muryanu, 48-40

Riga, 226004(SU)
Erfinder: TAURITIS, Gvido Voldemarovich
ul. Rigas, 33 Rizhsky raion
Latviiskaya SSR s.Salaspils, 229021(SU)
Erfinder: POLEVOI, Leonard Georgievich
ul. Mosfilmovskaya, 39-34
Moscow, 117330(SU)
Erfinder: PEREKALIN, Vsevolod Vasilievich
ul. Voskova, 2-5
Leningrad, 197198(SU)
Erfinder: NOVIKOV, Boris Mikhailovich
ul. Kuznetsovskaya, 4-28
Leningrad, 196128(SU)
Erfinder: GUTMANIS, Andris Ekabovich
ul. Lachplesha, 35-36
Riga, 226011(SU)
Erfinder: ZAMAKH, Vladimir Petrovich
ul. Mendeleeva, 4-87
Latviiskaya SSR Olaine, 229014(SU)
Erfinder: PENKE, Ilmar Kharievich
ul. Lenina, 313-67
Riga, 226037(SU)

(74) Vertreter: von Füner, Alexander, Dr. et al
Patentanwälte v. Füner, Ebbinghaus, Finck
Mariahilfplatz 2 & 3
D-8000 München 90(DE)

(54) VERFAHREN ZUR HERSTELLUNG VON 4-PHENYL-2-PYRROLIDON-1-ESSIGSÄURE AMID.

(57) Die Erfindung bezieht sich auf organische Chemie. Das Verfahren zur Herstellung von 4-Phenyl-2-pyrrolidon-1-essigsäureamid besteht darin, dass man das 4-Phenyl-2-pyrrolidon mit überschüssigem Alkali im Medium eines aprobischen Lösungsmittels bei einer Temperatur von 70 bis 130° C umsetzt, das angefallene N-substituierte Derivat des 4-Phenyl-2-pyrrolidons man mit Halogenes-

sigsäureester mit anschliessender Umsetzung des anfallenden Produktes mit dem wässerigen Ammoni-ak und Ausscheidung des Endprodukt es umsetzt.

Das herzustellende 4-Phenyl-2-pyrrolidon-1-essigsäureamid weist krampflösende Wirkung auf.

# VERFAHREN ZUR HERSTELLUNG VON
# 4-PHENYL-2-PYRROLIDON-1-ESSIGSÄURE AMID

## Gebiet der Technik

Die vorliegende Erfindung bezieht sich auf organische Chemie und insbesondere auf ein Verfahren zur Herstellung von 4-Phenyl-2-pyrrolidon-1-essigsäure amid.

## Vorhergehender Stand der Technik

Bekannt sind Verfahren zur Herstellung von 4-Phenyl-2-pyrrolidon-1-essigsäureamid, das krafmpflösende Wirkung aufweist. Bekannt ist, beispielsweise, ein Verfahren /Chemisch-pharmezeutisches Journal Nr. 11, 1980, "Meditsina", (Moskau), Seiten 43-48), das in der Bearbeitung des 4-Phenyl-2-pyrrolidons im Medium des absoluten Dioxans mit Natriumhydrid und mit Diäthylester der Bromessigsäure besteht. Der auf diese Weise gewonnene Diäthylester der 4-Phenyl-2--pyrrolidon-1-essigsäure mit einer Ausbeute von 52,7%, der in Vakuumdestillation gereinigt wird, wird weiterhin mit gasförmigem Ammoniak im Methanolmedium unter Erwärmung aminiert.

In dem genannten Veffahren zur Herstellung 4-Phenyl-2-pyrrolidon-1-essigsäureamid in seiner ersten Stufe verwendet man das im Gebrauch gefährlich Natriumhydrid als Ausgangsverbindung und in der zweiten Stufe das toxische Methanol. All das kompliziert die Technologie der Herstellung des Endproduktes.

Bekannt ist ebenfalls ein Verfahren zur Herstellung von Amid der 4-Phenyl-2-pyrrolidon-1-essigsäure (SU, A, 1265191), das darin besteht, dass man das 4-Phenyl-2-pyrrolidon mit silylierenden Reagensmitteln (Trimehhylhalogensilan bzw. Hexamethylsilazan) behandelt. Das hergestellte 1-Trimethylsilyl-4-phenyl--2-pyrrolidon setzt man der Umsetzung mit Bromessigsäure ester bei einer Temperatur von 130 bis 175°C mit gleichzeitiger Abdestillation des Trimethylbromsilans aus dem Gemisch aus. Die angfefallenen Ester führt man ins Amid mittels Ammonolyse mit wässrigem Ammoniak über.

Das genannte Verfahren zur Herstellung von 4-Phenyl--2-pyrrolidon—1-essigsäureamid gibt die Möglichkeit, in der Stufe der Gewinnung von Estern das im Gebrauch gefährliche Natriumhydrid und in der Stufe der Gewinnung von Amid das toxische Methanol auszuschliessen.

Das genannte Verfahren unter Einsatz von siliziumorganischen Verbindungen zeichnet sich jedoch durch Kompliziertheit der technologischen Prozessführung aus, es besteht aus drei Stufen und verlangt die Reinigung von Zwischenprodukten in jeder Stufe.

Offenbarung der Erfindung

Der Erfindung liegt die Aufgabe zugrunde, durch Veränderung der technologischen Arbeitsgänge die Technologie der Prozessführung zu vereinfachen und die Ausbeute an Endprodukt zu steigern.

Die Aufgabe wurde dadurch gelöst, dass im Verfahren zur Herstellung von 4-Phenyl-2-pyrrolidon-1-essigsäureamid, das die Entstehung aus dem 4-Phenyl-2-pyrrolidon seines N-substituierten Derivates, die Umsetzung des N-substituierten Derivates des 4-Phenyl-2-pyrrolidons mit Halogenessigsäure ester, anschliessende Umsetzung des angfefallenen Produktes mit wässerigem Ammoniak und die Isolierung des Endproduktes vorsieht, erfindungsgemäss, die Bildung aus dem 4-Phenyl-2-pyrrolidon seines N-substituierten Derivates durch Umsetzung des 4-Phenyl--2-pyrrolidons mit dem Alkaliüberschuss im Medium eines aprofischen Lösungsmittels bei einer Temperatur von 70 bis 130°C erfolgt. Für die Steigerung der Ausbeute an Endprodukt soll zweckmässigerweise als Alkali Kalium- oder Natriumhydroxid verwendet werden. Mit dem Ziel der Erhöhung der Ausbeute und der Vereingachung der Technologie der Prozessführung sollen zweckmässigerweise als aprobischen.Lösungsmittel Dimethylsulfoxid oder sein Gemisch mit Toluol oder Bensol, oder mit Dimethylester des Diäthylenglykols verwendet werden.

Als Halogenessigsäureester soll vorzugsweise Äthylchlorazetat verwendet werden.

Das erfindungsgemässe Verfahren ist im Vergleich zu den bekannten Verfahren einstufig, lässt sich unter grosstechnischen Bedingungen leicht realisieren. Die Ausbeute an Endprodukt erreicht 79%, berechnet auf Ausgangsprodukt.

Beste Ausführungsvariante der Erfindung

Das erfindungsgemässe Verfahren wird wie folgt realisiert.

Gemisch des 4-Phenyl-2-pyrrolidons mit Alkaliüberschuss (als solche wird, beispielsweise, Kalium- oder Netriumhydroxid genommen) erwärmt man bei einer Temperatur von 70 bis 130°C in Medium eines Aprotonlösungsmittels. Als Aprotonlösungsmittel soll vorzugsweise Dimehtylsulfoxid oder sein Gemisch mit Toluol oder Benzol, oder mit Dimethylester des Diäthylenglykols verwendet werden. Die Erwärmung erfolgt unter intensivem Vermischen bis zum Erzielen einer vollständigen Dispergierung von Alkali. Dann wird das Reaktionsgemisch mit Chloressigsäureester bearbeitet, mit Wasser verdünnt, mit Benzol estrahiert und eingedampft. Der Rückstand wird der Umsetzung mit einer konzentrierten Lösung des wässerigen Ammoniaks bei Raumtemperatur ausgesetzt. Das ausgeschiedene Endprodukt wird abgefiltert.

Die Ausbeute an Endprodukt beträgt bis zu 79 Masse%.

Zur besseren Erläuterung der vorliegenden Erfindung werden Nachstehende konkrete Beispiele für die Realisierung des angemeldeten Verfahrens angeführt.

Beispiel 1

Ein Gemisch aus 170 g (1,04 Mol) 4-Phenyl-2-pyrrolidon, 148 g (2,65 Mol) granuliertem Kaliumhydroxid und 1 liter Dimethylsulfoxid erwärmt man unter Vermischen am sieden en Wasserbad innerhalb von 7 Stunden. Der heissen Lösung (70°C) setzt man 323 g (2,65 Mol) Äthylchlorazetat innerhalb von 0,5 Stunden zu. Die Temperatur des Gemisches bringt man auf die Raumtemperatur, setzt man ihm 3 Liter Wasser und extrahiert man dreimal mit Benzol zu je 400 ml. Die Benzolauszüge dampft man ein und dem Rückstand setzt man unter Vermischen 500 ml 25%gens wässeriges Ammoniak zu. Nach 24 Stunden wird der ausgefallene Niederschlag abgefiltert, mit Wasser gewaschen, getrocknet und man erhält 165 g (72 Masse%) 4-Phenyl-2-pyrrolidon-1-essigsäureamid mit einem Schmelzpunkt von 130 bis 131°C (Wasser).

Beispiel 2

Ein Gemisch aus 80,5 g (0,5 Mol) 4-Phenyl-2-pyrrolidon, 70 g (1,25 Mol) granuliertem Kaliumhydroxid und 0,5 Liter Dimehtylsulfoxid erwärmt man unter Vermischen innerhalb von 12 Stunden an einem Ölbad bei einer Temperatur von 105°C. Der heissen Lösung (70°C) setzt man 153 g (1,25 Mol) Äthylchlorazetat zu, die Temperatur bringt man auf die Raumtemperatur, setzt man ihr 1,5 Liter Wasser zu und extrahiert man drei Mal mit Benzol zu je 200 ml. Die Benzolauszüge werden eingedampft und dem Ruckstand werden unter Vermischen 250 ml des 25%gen wässerigen Ammoniak zugesetzt, in 24 Stunden wird der ausgefallene Niederschlag abgefiltert, mit Wasser gewaschen und getrocknet. Man erhält 86 g (79 Masse%) von der 4-Phenyl-2-pyrrolidon-1-essigsäureamid mit einem Schmelzpunkt von 130 bis 131,5°C (Wasser).

Beispiel 3

Ein Gemisch aus 40,3 g (0,25 Mol) von 4-Phenyl-2-pyrrolidon, 30 g (0,75 Mol) granuliertem Natriumhydroxid, 200 ml Dimethylsulfoxid und 40 ml Dimethylester des Diäthylenglykols erwärmt man an einem Ölbad (130°C) unter Vermischen bis zum vollständigen Verschwinden von Granalien des Natriumhydroxids. Der heissen Lösung setzt man 92 g (0,75 Mol) Äthylchlorazetat zu. Nach 24 Stunden setzt man dem Reaktionsgemisch 0,5 Liter Wasser und extrahiert man drei Mal mit je 100 ml Benzol. Die Benzolauszüge werden eingedampft und dem Rückstand werden unter Vermischen 150 ml des 25%gen wässerigen Ammoniaks zugesetzt, in einem Tag wird der ausgefallene Niederschlag abgefiltert, mit Wasser gewaschen und getrocknet. Man erhält 27,5 g (51 Masse%) der 4-Phenyl--2-pyrrolidon-1-essigsäureamid mit einem Schmelzpunkt von 130,5 bis 131,5°C (Wasser).

Beispiel 4

Ein Gemisch aus 80,5 g (0,5 Mol) 4-Phenyl-2-pyrrolidon, 70 g (1,25 Mol) granuliertem Kaliumhydroxid, 400 ml Dimethylsulfoxid und 150 ml Toluol erwärmt man an einem Ölbad (130°C) unter Vermischen innerhalb von 4 Stunden. Der heissen Lösung setzt man 153 g (1,25 Mol) Äthylchlorazetat zu. Nach 24 Stunden werden dem Gemisch 1,5 l Wasser zugesetzt und es wird drei Mal mit je 200 ml Benzol estrahiert. Die Benzolauszüge werden eingedampft und dem Rückstand werden unter Vermischen 250 ml des 25%gen wässerigen Ammoniaks zugesetzt. In einem Tag wird der ausgefallene Niederschlag abgefiltert, mit Wasser gewaschen und getrocknet. Man erhält 60 g (55 Masse%) 4-Phenyl-2-pyrrolidon-1-essigsäureamid mit einem Schmelzpunkt von 130 bis 131°C (Wasser).

Beispiel 5

Ein Gemisch aus 80,5 g (0,5 Mol) 4-Phenyl-2-pyrrolidon, 70 g (1,25 Mol) granuliertem Kaliumhydroxid, 400 ml Dimethylsulfoxid und 200 ml Benzol erwärmt man an einem Ölbad (80°C) unter Vermischen bis zur vollständigen Auflösung der Granalien des Kaliumhydroxids. Der heissen Lösung setzt man 153 g (1,25 Mol) Äthylchlorazetat zu. Nach 24 Stunden setzt man dem Gemisch 1 Liter Wasser zu und extrahiert man drei Mal mit je 200 ml Benzol. Die Benzolauszüge werden eingedampft und dem Rückstand werden unter Vermischen 250 ml des 25%gen wässerigen Ammoniaks zugesetzt. In einem Tag wird der ausgefallene Niederschlag abgefiltert, mit Wasser gewaschen und getrocknet. Man erhält 58 g (53 Masse%) 4-Phenyl--2-pyrrolidon-1-essigsäureamid mit einem Schmelzpunkt von 131 bis 131,5°C (Wasser).

Beispiel 6

Ein Gemisch aus 40,3 g (0,25 Mol) von 4-Phenyl--2-pyrrolidon, 28 g (0,5 Mol) granuliertem Kaliumhydroxid und 100 ml Dimehtylsulfoxid erwärmt man am einem Ölbau (70°C) unter Vermischen bis zum Verschinden der Granalien des Kaliumhydroxids. Der heissen Lösung setzt man tropfenweise 83,5 g (0,5 Mol) Äthylbromazetat zu. Nach 24 Stunden setzt man dem Gemisch 0,5 l Wasser zu und extrahiert man drei Mal mit je 100 ml Benzol. Die Benzolauszüge werden vereint, eingedampft und dem Rückstand werden unter Vermischen 150 ml des 25%gen wässerigen Ammoniaks zugesetzt. In einem Tag wird der ausgefallene Niederschlag abgefiltert, mit Wasser gewaschen und getrocknet. Man erhält 35,5 g (65 Masse%) 4-Phenyl-2-pyrrolidon-1-essigsäureamid mit einem Schmelzpunkt von 130 bis 131°C (Wasser).

Beispiel 7

Ein Gemisch aus 0,832 kg (5 Mol) von 4-Phenyl--2-pyrrolidon, 0,806 kg (12,5 Mol) Kaliumhydroxid, 3,28 l Dimethylsulfoxid und 5 l Benzol wird unter Vermischen mit einem Wasserabscheidungsmittel innerhalb von 10 Stunden gekockt. Dem heissen Gemisch setzt man 1,62 kg (13 Mol) Äthylchlotazetat zu. In 2 Stunden wird das Reaktionsgemisch den 10 Liter Wasser gegossen, die obere Schicht wird abgeschieden und die untere mit 3 l Benzol extrahiert. Die vereinten Benzolauszüge werden eingedampft und dem Rückstand werden 4,2 l des wässerigen Ammoniaks zugesetzt, in einem Tag wird der ausgefallene Niederschlag abgefiltert, mit Wasser gewaschen und getrocknet. Man erhält 530 l (49 Masse%) 4-Phenyl-2-pyrrolidon-1-essigsäureamid mit einem Schmelzpunkt von 130 bis 133°C (Wasser).

Gewerbliche Anwendbarkeit

Das gemäss dem angemeldeten Verfahren herzustellende 4-Phenyl-2-pyrrolidon-1-essigsäureamid weist eine krampflösende Aktivität auf und findet Anwendung in der Medizin.

## PATENTANSPRÜCHE

1. Verfahren zur Herstellung 4-Phenyl-2-pyrrolidon-
-1-essigsäureamid, das die Umsetzung von 4-Phenyl-2-pyrrolidon zu seinem N-substituierten Derivat, die Umsetzung
des N-substituierten Derivats -des 4-Phenyl-2-pyrroli-
dons- mit Halogenessigsäureester, die anschliessende
Umsetzung des angefallenen Produktes mit wässerigem
Ammoniak und die Abscheidung des Endproduktes vorsieht,
dadurch g e k e n n z e i c h n e t , dass die Umsetzung
des 4-Phenyl-2-pyrrolidon zu seinem N-substituierten
Derivat durch Umsetzung des 4-Phenyl-2-pyrrolidons
mit einem Alkaliüberschuß im Medium eines aprotischen
Lösungsmittels bei einer Temperatur von 70 bis 130°C
erfolgt.

2. Verfahren nach Anspruch 1, dadurch g e k e n n -
z e i c h n e t , dass man als Alkali Kalium- oder
Natriumhydroxid verwendet.

3. Verfahren nach Anspruch 1 bis 2, dadurch g e -
k e n n z e i c h n e t , dass man als aprotische Lösungsmittel Dimethylsulfoxid oder sein Gemisch mit Toluol oder Benzol, oder mit Dimethylester des Diethylenglykols verwendet.

4. Verfahren nach Anspruch 1 bis 3, dadurch
g e k e n n z e i c h n e t , dass man als Ester der
Halogenessigsäure ethylchlorazetat verwendet.

# INTERNATIONAL SEARCH REPORT

International Application No  PCT/SU 88/00083

## I. CLASSIFICATION OF SUBJECT MATTER (if several classification symbols apply, indicate all) *

According to International Patent Classification (IPC) or to both National Classification and IPC

Int. Cl.$^4$ - C 07 D   207/27

## II. FIELDS SEARCHED

### Minimum Documentation Searched [7]

| Classification System | Classification Symbols |
|---|---|
| Int.Cl.$^4$ | C 07 D   207/24,   207/26, 207/27 |

### Documentation Searched other than Minimum Documentation
### to the Extent that such Documents are Included in the Fields Searched [8]

## III. DOCUMENTS CONSIDERED TO BE RELEVANT [9]

| Category * | Citation of Document, [11] with indication, where appropriate, of the relevant passages [12] | Relevant to Claim No. [13] |
|---|---|---|
| A | SU, AI, 1265191 (2-i Moskovsky ordena Lenina Gosudarstvenny meditsinsky institut im N.I. Pirogova et al.) 23  October 1986 (23.10.86), (cited in the description) | 1-4 |
| A | SU, AI, 1265192 (2-i Moskovsky ordena Lenina Gosudarstvenny meditsinsky institut im N.I. Pirogova et al.), 23 October 1986 (23.10.86), see the claims | 1-4 |
| A | Khimiko-farmatsevtichesky zhurnal, tom XIV, No.11, 1980 (Meditsina, Moscow), O.M. Glozman et al., "Sintez i protivosudorozhnaya aktivnost amidov 4-fenil-pirrolidon-2-uksusnoi-1 kisloty," see pages  43-48 | 1-4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 22 November 1988  (22.11.88) | 13. January 1989  (13.01.89) |

| International Searching Authority | Signature of Authorized Officer |
|---|---|
| ISA/SU | |

Form PCT/ISA/210 (second sheet) (January 1985)